# EUROPEAN PATENT SPECIFICATION

(11) **EP 2 097 126 B1**
(45) Date of publication and mention of the grant of the patent: **22.05.2019**
(21) Application number: 07811026.9
(22) Date of filing: 02.08.2007
(51) Int. Cl.: A61M 5/34, A61M 5/32, A61M 5/31, A61M 5/315

(54) **DETACHABLE NEEDLE SYRINGE HAVING REDUCED DEAD SPACE**
SPRITZE MIT ABNEHMBARER NADEL MIT VERMINDERTEM TOTVOLUMEN
SERINGUE À AIGUILLE DÉTACHABLE DOTÉ D'UN ESPACE MORT RÉDUIT

(30) Priority: 03.08.2006 US 462083
(43) Date of publication of application: 09.09.2009
(73) Proprietor: Becton, Dickinson and Company, Franklin Lakes, NJ 07417-1880 (US)
(72) Inventor: D'ARRIGO, Christina, Joy, Hoboken, NJ 07030 (US); SCHILLER, Eric, Kinnelon, NJ 07405 (US)
(74) Representative: dompatent von Kreisler Selting Werner - Partnerschaft von Patent- und Rechtsanwälten mbB
(86) International application number: PCT/US2007/017275
(87) International publication number: WO 2008/019035

(56) References cited:
- EP-A1- 0 022 576
- WO-A1-92/09320
- WO-A1-99/62579
- WO-A1-2006/020953
- CH-A- 380 305
- US-A- 5 002 538
- US-A- 5 782 803
- US-A1- 2002 173 753
- US-B1- 6 171 285

## Description

### FIELD OF THE INVENTION

The present invention relates to syringes with removable needle assemblies. More particularly, the present invention relates to a syringe and needle assembly having structure for consistently minimizing dead space in a syringe barrel while containing an effective seal between the syringe barrel and the needle assembly.

### BACKGROUND OF THE INVENTION

Syringe assemblies designed for use with replaceable needle assemblies usually contain a luer slip or locking luer type fitting for securing the needle assembly to the syringe barrel. These connections rely on an elongate frusto-conically shaped syringe barrel tip which frictionally engages a frusto-conically shaped passageway in a needle hub. The relatively small acute angle of the syringe tip, measured from the longitudinal axis of the syringe barrel, provides an excellent seal between the syringe tip and the hub. However, slight variations in the angle or diameter of the tapered surfaces of the syringe barrel and/or the needle hub produce large variations in the relative position of the needle hub with respect to the end of the barrel and, therefore, variations in dead space. The space inside the barrel tip and between the end of the barrel tip and the end of the cavity in the hub constitutes a dead space containing liquid that cannot be delivered by the syringe.

In the case of expensive medications and multi-patient immunization programs, medication lost in the dead space in single dose, multidose and prefilled syringes can become costly. This is especially true in immunization programs involving thousands of people. Controlling dead space to a minimum may result in more people being immunized with the same amount of medication provided for the program.

The prior art also teaches a flat seal, perpendicular to the longitudinal axis of the syringe barrel which mates with a flat seal on the needle hub for use with a threaded needle assembly and barrel engagement structure. The flat seal then greatly reduces the portion of the dead space attributable to variations in barrel and hub tolerances. However, imperfections on either or both of the mating flat surfaces can result in a leaky seal which allows medication to leave the syringe during use.

WO 1999/62579 describes a syringe and detachable needle assembly as defined within the preamble of claim 1.

Although the prior art teaches many syringe barrel and needle hub connecting structures, there is still a need for a low dead space syringe which is easy to manufacture and provides consistently low dead space from syringe to syringe and an effective seal between the needle assembly and the syringe barrel.

### SUMMARY OF THE INVENTION

A syringe and detachable needle assembly having reduced dead space features as defined in claim 1 includes an elongate syringe barrel having a longitudinal axis, an inside surface defining a chamber for retaining fluid, an open proximal end, an open distal end including a collar and a distally facing annular, preferably flat surface projecting inwardly from the collar. A portion of the collar includes a cylindrically shaped, axially aligned circular sidewall. A needle assembly includes a hub and a cannula. The hub includes a body portion having a proximal end, a distal end, a conduit therethrough, a proximally facing annular, preferably flat surface on the body portion contacting the annular surface of the barrel forming a primary seal between the hub and the barrel. The hub further includes an outwardly projecting annular sealing ring engaging the cylindrically shaped axially aligned portion of the sidewall of the collar forming a secondary seal between the hub and the barrel. The needle assembly further includes a cannula having a distal end, a proximal end and a lumen therethrough. The proximal end of the cannula is connected to the distal end of the hub so that the lumen of the cannula is in fluid communication with the chamber in the barrel. Structure is also provided for releasably engaging the hub to the collar.

The syringe assembly may also include an elongate plunger rod having a proximal end, a distal end and a stopper at the distal end of the plunger rod. The stopper is positioned in fluid tight engagement with the inside surface of the barrel for displacing fluid from the chamber through the cannula by relative motion of the plunger rod with respect to the barrel. The stopper may include a distally facing projection for partially occluding the conduit in the hub when the stopper is in its distal most position inside the barrel.

The outwardly projecting annular sealing ring can be any structure which is resilient enough to deflect upon installation of the needle assembly to the barrel so that the annular sealing ring deflects upon contact with the cylindrically shaped, axially aligned circular sidewall portion of the collar. The annular sealing ring can be an elastomeric o-ring preferably positioned in an annular groove in the hub. The o-ring preferably has a circularly shaped cross-section. The annular sealing ring can also be a tapered projection having a base adjacent to the body portion of the hub and a free end wherein the tapered projection is wider at its base than at its free end. The annular sealing ring may also be a radially outwardly projecting cantilever shaped projection. This projection may be positioned between two annular grooves on the hub. Also, the annular sealing ring may be integrally formed with the body portion of the hub.

An elongate hollow needle shield having a distal end and an open proximal end can be removably engaged to the hub so that the needle shield covers the cannula.

A needle assembly may include a hub and cannula that are integrally formed with thermoplastic material. The cannula may also be formed of metal such as stainless steel and mechanically connected to the hub. The cannula can have a sharp distal tip or a blunt distal tip. The structure for releasably engaging the hub to the collar may include at least one thread on the collar or on the hub. The at least one thread may be right-handed thread and/or a multiple lead thread.

The structure for releasably engaging the hub to the collar includes at least one thread engaging projection on the hub or the collar and at least one thread on the other of the hub or the collar.

### BRIEF DESCRIPTION OF THE DRAWINGS

Fig. 1 is an exploded view illustrating a prior art syringe and needle assembly.
Fig. 2 is a partial cross-sectional view of the prior art needle assembly of Fig. 1 connected to the prior art syringe of Fig. 1.
Fig. 3 is a prospective view of the syringe and detachable needle assembly of the present invention.
Fig. 4 is an exploded view of the syringe and detachable needle assembly of the present invention.
Fig. 5 is a side elevational view of the needle assembly, with needle shield attached, of the present invention.
Fig. 6 is a cross-sectional view of the needle assembly of Fig. 5 taken along lines 6-6.
Fig. 7 is a partial enlarged cross-sectional view of the distal end of the barrel of Fig. 4.
Fig. 8 is a partial cross-sectional view illustrating the needle assembly connected to the syringe barrel.
Fig. 9 is an alternative embodiment of the needle assembly of the present invention having, an integrally formed sealing ring.
Fig. 10 is a side elevational view of another embodiment of the needle assembly of present invention.
Fig. 11 is an enlarged cross-sectional view of the distal end of the barrel for use with a needle assembly of Fig. 10.
Fig. 12 is a partial cross-sectional view illustrating the needle assembly of Fig. 10 connected to the barrel of Fig. 11.

### DETAILED DESCRIPTION

While this invention is satisfied by embodiments in many different forms, there are shown in the drawings and will herein be described in detail, preferred embodiments of the invention with the understanding that the present disclosure is to be considered exemplary of the principles of the invention and not intended to limit the invention to the embodiments illustrated. The scope of the invention will be measured by the appended claims and their equivalents.

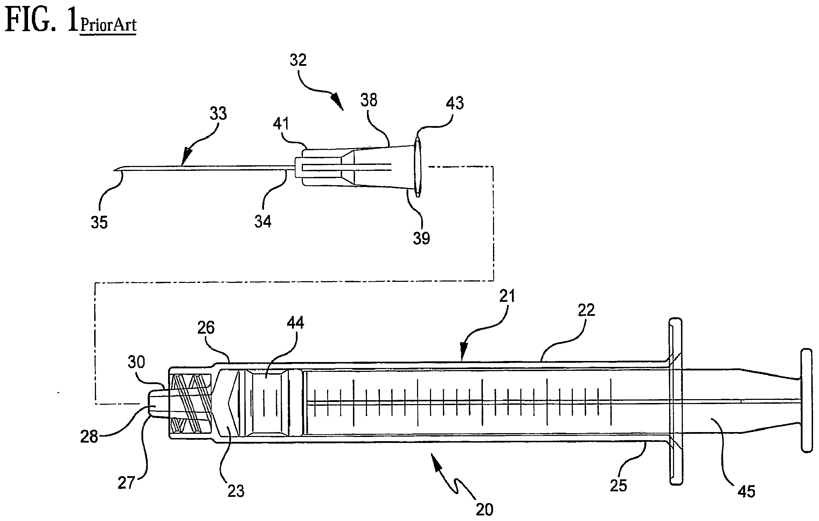

Referring to Figs. 1-2, a prior art syringe assembly 20 includes a barrel 21 having an elongate body 22 defining a chamber 23 for retaining fluid. The barrel includes an open proximal end 25 and distal end 26 including a tip 27 having a passageway 28 therethrough in fluid communication with the chamber. The distal end of the barrel may also include a locking luer-type collar 29 concentrically surrounding tip 27. The luer collar has an internal thread 31.

A prior art needle assembly 32 includes a cannula 33 having a proximal end 34, a distal end 35 and lumen 37 therethrough. A hub 38 includes an open proximal end 39 containing a cavity 40 and a distal end 41 attached to proximal end 34 of the cannula so that the lumen of the cannula is in fluid communication with the cavity in the hub. The needle assembly is removably attached to the syringe through frictional engagement of the interior surface of the cavity in the hub and the exterior surface of tip 27. The hub also includes radial projections 43 on its proximal end for engaging thread 31 to hold the needle assembly securely to the barrel. The prior art syringe assembly further includes a resilient stopper 44 connected to an elongate plunger rod 45.

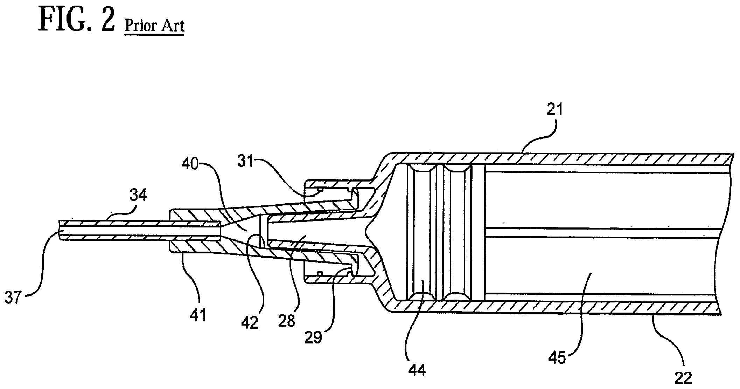

Liquid in a syringe barrel can experience very high pressures during the injection process. This is especially true in the case of high viscosity medications and/or needle cannula having a lower diameter lumen. To seal the interface between the needle hub and the syringe against these high pressures, a frusto-conically shaped engagement surface 30 on tip 27 frictionally engages a frusto-conically shaped engagement surface 42 in hub cavity 40. When the diameter of the engagement surface 42 is on the small end of its tolerance range and the diameter of the engagement surface 30 of the barrel tip is on the large end of its tolerance range, it can be seen that the barrel tip will not move as far into the cavity of the needle hub thus leaving more empty space in the hub cavity. Although the seal created by the frusto-conically shaped engagement surfaces is very effective, the components must be designed to accept all anticipated tolerance variations of the barrel tip and hub cavity so that the distance the barrel engages the hub cavity will vary throughout the tolerance range thus varying the amount of unoccluded space in the hub cavity. As can be seen in Fig. 2, the dead space includes the unoccluded space in the tip passageway 28, inherent in the luer slip design, and the unoccluded space in the hub cavity 40, partially necessitated by tolerance variations. The unoccluded space in the barrel tip is inherent in the design while the occluded space in the hub cavity will vary widely to the tolerance variation properties of the luer-type engagement. Accordingly, dead space is difficult to control in the prior art syringe using luer-type fittings.

Other prior art syringe assemblies have attempted to minimize and reduce the variability of dead space by providing frusto-conically shaped engagement surfaces which are at much larger angles with respect to the longitudinal axis of the barrel to shorten the variation in the dead space based on tolerances. The best control of dead spaces can be achieved with a flat engagement surface positioned approximately at an angle 90° with respect to the longitudinal axis of the barrel. This configuration best controls dead space caused by tolerance variation, and can overcome inherent dead space in the luer slip barrel tip. However, imperfections on the mating surfaces or variations in the flatness of these surfaces can result in a seal between the hub and the barrel which may leak initially or over a period of time. Also, the quality of the seal is directly related to the amount of force applied to the hub while engaging it to the barrel.

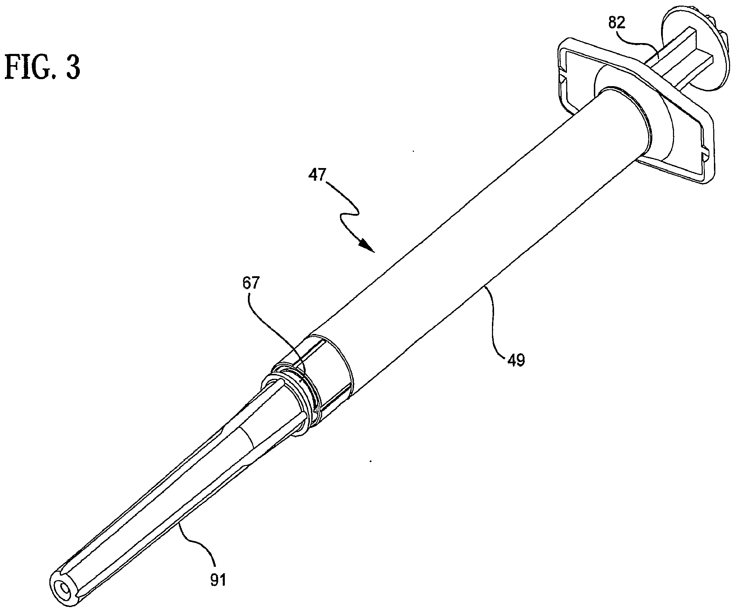

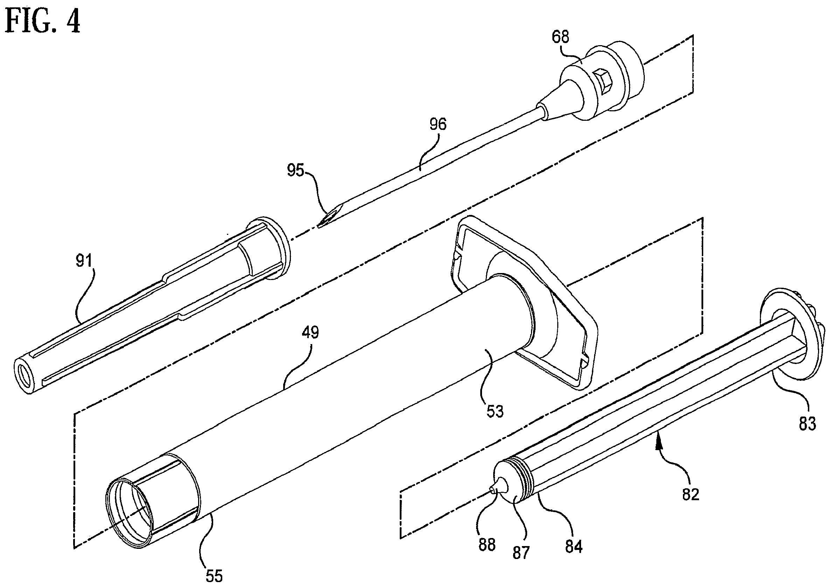

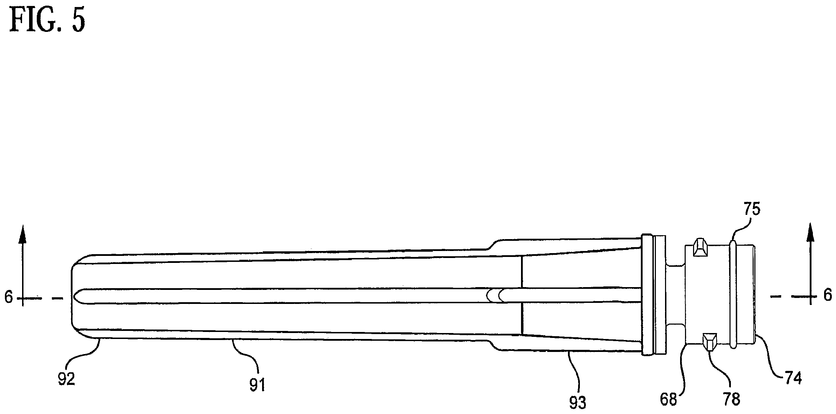

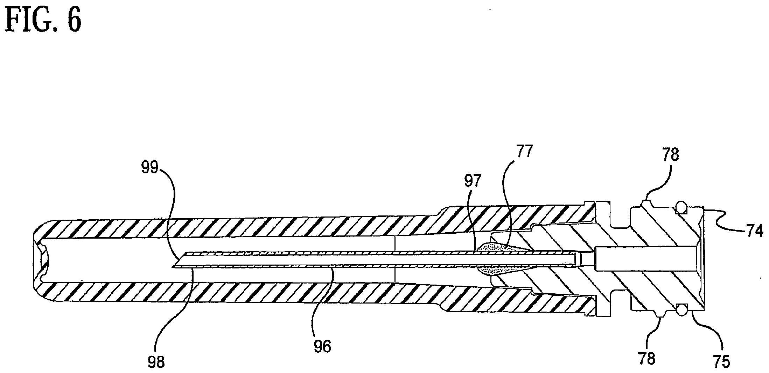

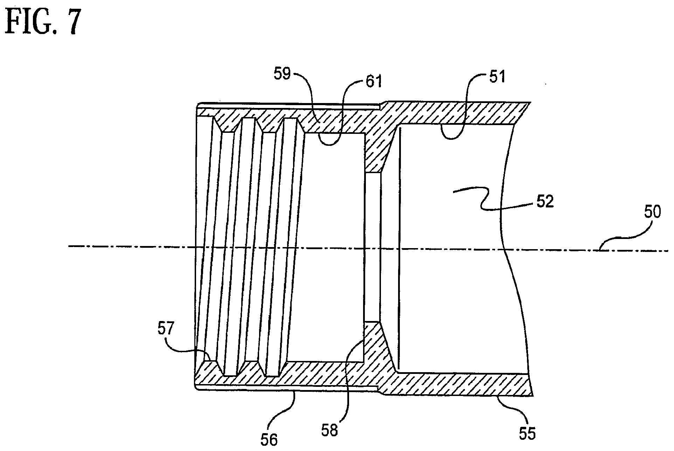

Referring to Figs. 3-8, a syringe and detachable needle assembly 47 of the present invention, having reduced dead space features includes an elongate syringe barrel 49 having a longitudinal axis 50, an inside surface 51 defining a chamber 52 for retaining fluid. Barrel 49 further includes an open proximal end 53, an open distal end 55 including a collar 56 having a needle hub engaging structure and a distally facing annular surface 58 projecting inwardly from the collar. Annular surface 58 is preferably flat. A portion of the collar includes a cylindrically shaped, axially aligned circular sidewall.

A needle assembly 67 includes a hub 68 having a body portion 69 including a proximal end 70, a distal end 71 and a conduit 73 therethrough. A proximally facing annular surface 74 on the body portion contacts the annular surface on the barrel forming a primary seal between the hub and the barrel. Annular surface 74 is preferably flat. The hub also includes an outwardly projecting annular sealing ring 75 sealingly engaging the circular sidewall of the collar forming a secondary seal between the hub and the barrel.

In this embodiment, the annular sealing ring is an o-ring contained in annular groove 76 around the body portion of the hub. As will be more fully explained hereinafter, the outwardly projecting annular sealing ring can be any structure which is resilient enough to deflect upon installation of the needle assembly to the barrel so that the annular sealing ring deflects upon contact with circular sidewall portion of the collar. These structures are all within the purview of the present invention. The o-ring and other structures for the annular sealing ring taught herein are merely representative of these many possibilities.

Needle assembly further includes a cannula 96 having proximal end 97, a distal end 98 preferably having a sharp distal tip 95 and a lumen 99 therethrough. The proximal end of the cannula is connected to the distal end of the hub so that the lumen is in fluid communication with the chamber in the barrel. The cannula may be integrally formed with the hub such as by injection molding using thermoplastic materials or separately formed, as in this embodiment, and attached to the hub. In this embodiment adhesive 77 is used to attach the cannula to the hub, and the cannula is made of metal, preferably stainless steel.

Structure for releasably engaging the hub to the collar including hub engaging structure on the collar and complimentary collar engaging structure on the hub is provided. In this embodiment structure for releasably engaging the hub and collar includes at least one thread 57 on an inside surface 61 of the circular shaped sidewall 59. The thread may be either right-handed or left-handed, and it also may be a multiple-lead thread. The hub includes at least one thread engaging the projection. There are two thread engaging projections 78, on opposite sides of the hub.

The syringe assembly may also include an elongate plunger rod 82 having a proximal end 83, a distal end 84 and a stopper 87 at the distal end of the plunger rod. A stopper is slidably positioned in fluid tight engagement with the inside surface of the barrel for displacing fluid from the chamber through the cannula by relative motion of the plunger with respect to the barrel. The stopper desirably includes a distally facing projection 88 for partially occluding the conduit in the hub when the stopper is in its distal most position inside the barrel to further reduce dead space.

The needle assembly may further include an elongate, hollow needle shield 91 having a distal end 92 and open proximal end 93 removably engaged to the hub so that the needle shield covers the cannula.

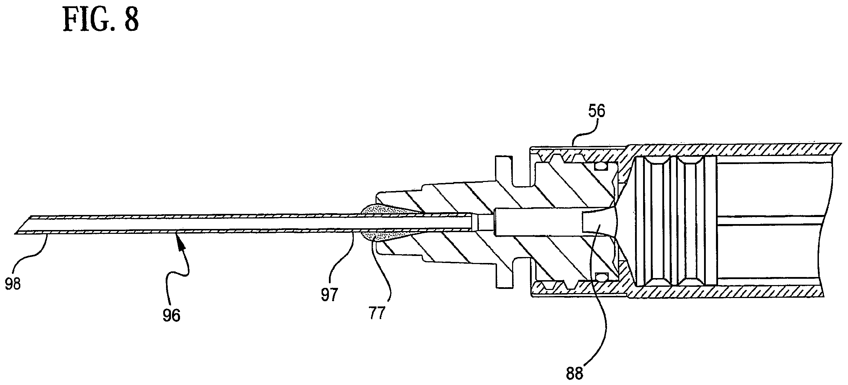

The needle assembly maybe installed or removed from the barrel by grasping the needle shield and rotating the hub in the appropriate direction to install or disengage the needle assembly. During installation, rotation of the hub will cause the needle engaging projections of the hub to follow the thread until proximally facing annular surface 74 of the hub contacts distally facing annular surface 58 of the barrel to form primary seal between the hub and the barrel. In this position, outwardly projecting annular sealing ring 75 is deflected as illustrated in Fig. 8 to form a secondary seal between the hub and the barrel. The secondary seal is a backup seal in the event that there are imperfections or other discontinuities on the annular surfaces or other factors that compromise their sealing ability or in the event that the user fails to provide enough rotational torque while connecting the hub to the barrel to adequately engage the primary seal.

It should be noted that the proximally facing annular surface 74 when it is flat on the hub and the distally facing annular surface 58 when it is flat on the barrel need not be aligned exactly at 90° from the longitudinal axis. A broad range of angles will work with the angles of 88° to 92° being desired and 90° being preferred.

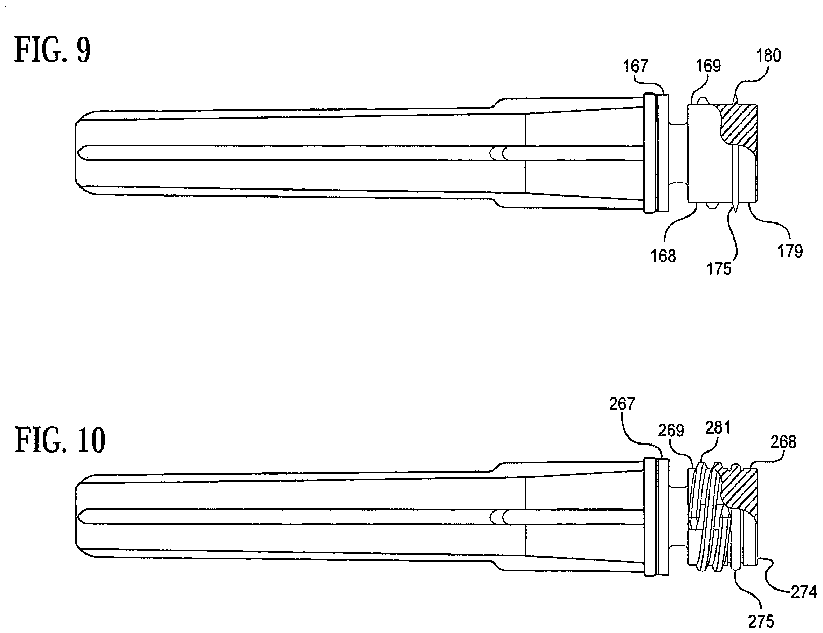

Fig. 9 illustrates an alternative embodiment of the needle assembly of the present invention. In this embodiment, needle assembly 167 performs similarly to the needle assembly of Figs. 3-8. In this embodiment, hub 168 has an outwardly projecting annular sealing ring 175 which is integrally molded with body portion 169 of the hub. The annular sealing ring is a tapered projection having a base 179 adjacent to the body portion and a free end 180. Tapered projection in this embodiment is wider at its base than at its free end. Projection functions similarly to the projection in the embodiment of Figs. 3-8.

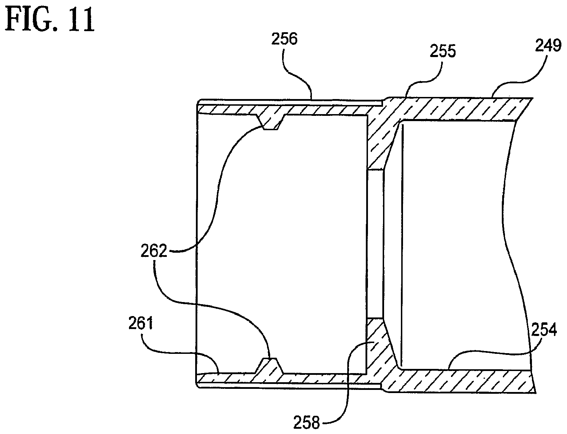

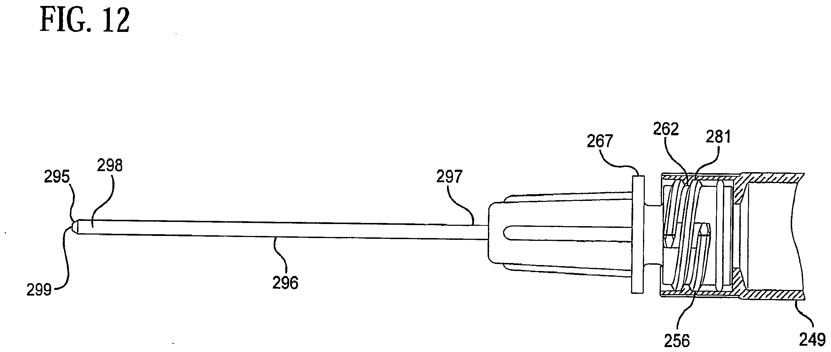

Figs. 10,11 and 12 illustrate another alternative embodiment of the syringe assembly of the present invention. In this embodiment, needle assembly 267 includes a hub 268 having a body portion 269 wherein the body portion includes at least one thread 281, an outwardly projecting cantilever shaped annular sealing ring 275 positioned between annular grooves 276 and a proximally facing annular flat surface 274. Needle assembly 267 further includes a cannula 296 having a proximal end 297, a distal end 298, preferably having a blunt distal tip 295 and a lumen 299 therethrough. The proximal end of the cannula is connected to the distal end of the hub so that the lumen is in fluid communication with the chamber and the barrel. In this embodiment, the cannula is integrally formed with the hub using thermoplastic materials and an injection molding process. A blunt tip is well suited for use with fluid transfer devices having a split septum and other fluid transfer devices designed for accepting blunt cannula.

An elongate barrel 249 includes an inside surface 251 defining a chamber 252 and an open distal end 255 and a collar 256. The collar includes an inside surface 261 having at least one thread engaging projection. In this embodiment there are two thread engaging projections 262 facing inwardly for engaging thread 281 on hub 268. The components of this embodiment function similarly to the embodiment of Figs. 3-8. In this embodiment, proximally facing annular flat surface 274 on the hub engages distally facing annular flat surface 258 in the barrel to form the primary seal between the hub and the barrel. Cantilever shaped outwardly facing annular sealing ring 275 will be deflected by inside surface 261 during installation to provide a secondary seal between the hub and the barrel.

There are many ways to configure the hub and the collar of the barrel including a hub having an additional skirt which surrounds the collar of the syringe barrel. With this construction, the outside of the collar may include at least one thread or at least one thread-engaging projection while the inside of the hub skirt will include the other of a thread or at least one thread-engaging projection. The engagement between the hub and the collar, in all configurations, can include a thread or thread-like structure on the hub and a thread or thread-like structure on the collar. All of these structures are within the purview of the present invention as defined by the appended claims and the embodiments taught hereinabove are merely representative of these many possibilities.

## Claims

1. A syringe and detachable needle assembly (47) having reduced dead space features comprising:
an elongate syringe barrel (49) having a longitudinal axis (50), an inside surface (51) defining a chamber (52) for retaining fluid, an open proximal end (53), an open distal end (55) including a collar (56) and a distally facing annular surface (58) projecting inwardly from said collar, a portion of said collar having a cylindrically shaped, axially aligned circular side wall;
a needle assembly (67) including a hub (68) having a body portion (69) including a proximal end (70), a distal end (71), a conduit (73) therethrough, a proximally facing annular surface (74) on said body portion contacting said annular surface of said barrel forming a primary seal between said hub and said barrel, said hub further including an outwardly projecting annular sealing ring (75) sealingly engaging said circular side wall of said collar forming a secondary seal between said hub and said barrel, said needle assembly (67) further including a cannula (96) having a distal end (98), a proximal end (97) and a lumen (99) therethrough, said proximal end of said cannula being connected to said distal end of said hub so that said lumen is in fluid communication with said chamber; and
means for releasably engaging said hub to said collar including at least one thread (57) on an inside surface (61) of said collar or at least one thread on an outside surface of said hub (68),
**characterized by**
wherein distally facing annular surface (58) of the barrel is flat and proximally facing annular surface (74) of the hub is flat;
two thread engaging projections (78) on opposite sides of the hub (68);
wherein during installation, rotation of the hub (68) will cause the needle engaging projections (78) of the hub (68) to follow the thread until the proximally facing annular surface (74) of the hub (68) contacts distally facing annular surface (58) of the barrel (49) to form primary seal between the hub and the barrel;
the outwardly projecting annular sealing ring (75) is positioned between the threaded projections (78) and the proximal facing annular surface (74) of the hub;
and the needle assembly is installed or removed from the barrel by rotating the hub, and wherein the secondary seal is a backup seal in the event that the user fails to provide enough rotational torque while connecting the hub to the barrel to adequately engage the primary seal.

2. The syringe assembly of Claim 1 further including an elongate plunger rod (82) having a proximal end (83), a distal end (84) and a stopper (87) at said distal end of said plunger rod, said stopper being slidably positioned in fluid tight engagement with said inside surface of said barrel for displacing fluid from said chamber through said cannula by relative motion of said plunger rod with respect to said barrel.

3. The syringe assembly of Claim 2 wherein said stopper includes a distally facing projection (88) for partially occluding said conduit in said hub when said stopper is in its distal most position inside said barrel.

4. The syringe assembly of Claim 2 wherein said annular sealing ring (75) is an elastomeric o-ring and wherein said o-ring has preferably a circularly shaped cross-section.

5. The syringe assembly of Claim 1 wherein said annular sealing ring (75, 175) is a tapered projection having a base (179) adjacent to said body portion and a free end (180), said tapered projection being wider at said base than at said free end.

6. The syringe assembly of Claim 1 wherein said annular sealing ring (75, 175) is a radial, cantilever shaped projection (275) or wherein said annular sealing ring (275) is positioned between two annular grooves (276) or wherein said annular sealing ring is integrally formed with said body portion.

7. The syringe assembly of Claim 1 further including an elongate hollow needle shield (91) having a distal end (92) and an open proximal end (93) removably engaged to said hub so that said needle shield covers said cannula.

8. The syringe assembly of Claim 1 wherein said hub (68) and said cannula (96) are integrally formed of thermoplastic material.

9. The syringe assembly of Claim 1 wherein said cannula (96, 296) includes a blunt distal tip (295) or wherein said cannula is formed of metal.

10. The syringe assembly of Claim 1 wherein said at least one thread (57) is a righthand thread and/ or wherein said at least one thread is a multiple lead thread.

11. The syringe assembly of Claim 1 wherein said means for releasably engaging said hub to said collar includes at least one thread engaging projection (78) on said hub or at least one thread engaging projection on an inside surface of said collar.

12. A syringe and detachable needle assembly of claim 1 further comprising:
an elongate hollow needle shield (91) having a distal end (92) and an open proximal end (93) removably engaged to said hub so that said needle shield (91) covers said cannula; and
an elongate plunger rod (82) having a proximal end (83), a distal end (84) and a stopper (87) at said distal end of said plunger rod, said stopper (87) being slidably positioned in fluid tight engagement with said inside surface of said barrel (49) for displacing fluid from said chamber through said cannula (96) by relative motion of said plunger rod (82) with respect to said barrel (49).

## Patentansprüche

1. Spritze und abnehmbare Nadelvorrichtung (47) mit verminderten Totraummerkmalen mit:
einem verlängerten Spritzenzylinder (49) mit einer Längsachse (50), einer Innenfläche (51), die eine Kammer (52) zur Flüssigkeitsaufbewahrung ausbildet, einem offenen proximalen Ende (53), einem offenen distalen Ende (55), das einen Kragen (56) und eine distal ausgerichtete Ringfläche (58) aufweist, die vom Kragen ausgehend nach innen gerichtet ist, wobei ein Abschnitt des Kragens eine zylinderförmige, axial ausgerichtete ringförmige Seitenwand aufweist;
einer Nadelvorrichtung (67), die einen Ansatz (68) aufweist mit einem Körperabschnitt (69), der ein proximales Ende (70), ein distales Ende (71) und einen durch den Körperabschnitt verlaufenden Durchlass (73) aufweist, wobei eine proximal ausgerichtete Ringfläche (74) auf dem Körperabschnitt die Ringfläche des Zylinders kontaktiert, so dass eine Primärdichtung zwischen dem Ansatz und dem Zylinder entsteht, wobei der Ansatz ferner einen nach außen gerichteten ringförmigen Dichtring (75) aufweist, der abdichtend in die kreisförmige Seitenwand des Kragens eingreift, so dass eine Sekundärdichtung zwischen dem Ansatz und dem Zylinder entsteht, wobei die Nadelvorrichtung (67) ferner eine Kanüle (96) aufweist mit einem distalen Ende (98), einem proximalen Ende (97) und einem durch die Kanüle verlaufenden Lumen (99), wobei das proximale Ende der Kanüle mit dem distalen Ende des Ansatzes verbunden ist, so dass das Lumen fluidisch mit der Kammer verbunden ist, und Vorrichtungen zum lösbaren Eingreifen des Ansatzes in den Kragen mit mindestens einem Gewinde (57) an einer Innenfläche (61) des Kragens oder mindestens einem Gewinde an einer Außenfläche des Ansatzes (68),
**dadurch gekennzeichnet, dass**
die distal ausgerichtete Ringfläche (58) des Zylinders flach ist und die proximal ausgerichtete Ringfläche (74) des Ansatzes flach ist;
zwei Gewindeeingriffsvorsprünge (78) an gegenüberliegenden Seiten des Ansatzes (68) ausgebildet sind;
beim Zusammenbauen das Drehen des Ansatzes (68) dazu führt, dass die Eingriffsvorsprünge (78) des Ansatzes (68) für die Nadel dem Gewinde folgen, bis die proximal ausgerichtete Ringfläche (74) des Ansatzes (68) die distal ausgerichtete Ringfläche (58) des Zylinders (49) kontaktiert, um eine Primärdichtung zwischen dem Ansatz und dem Zylinder zu bilden;
der nach außen gerichtete ringförmige Dichtring (75) zwischen den Gewindeeingriffsvorrichtungen (78) und den proximal ausgerichteten Ringflächen (74) des Ansatzes positioniert ist; und
die Nadelvorrichtung eingesetzt oder aus dem Zylinder entfernt wird durch Drehen des Ansatzes, und wobei die Sekundärdichtung eine Backup-Dichtung ist für den Fall, dass der Benutzer beim Verbinden des Ansatzes mit dem Zylinder nicht genügend Drehmoment bereitstellt, um die Primärdichtung ausreichend einzugreifen.

2. Spritzenvorrichtung nach Anspruch 1, die ferner einen verlängerten Kolben (82) aufweist mit einem proximalen Ende (83), einem distalen Ende (84) und einem Stopfen (87) am distalen Ende des Kolbens, wobei der Stopfen verschiebbar positioniert ist in fluiddichter Verbindung mit der Innenfläche des Zylinders, um die Flüssigkeit aus der Kammer durch die Kanüle zu verdrängen mittels einer Relativbewegung des Kolbens zum Zylinder.

3. Spritzenvorrichtung nach Anspruch 2, wobei der Stopfen einen distal ausgerichteten Vorsprung (88) aufweist, um den Durchlass im Ansatz teilweise zu verschließen, wenn der Stopfen sich in seiner distalsten Position im Zylinder befindet.

4. Spritzenvorrichtung nach Anspruch 2, wobei der ringförmige Dichtring (75) ein Elastomer-O-Ring ist und wobei der O-Ring vorzugsweise einen kreisförmigen Querschnitt aufweist.

5. Spritzenvorrichtung nach Anspruch 1, wobei der ringförmige Dichtring (75, 175) ein spitz zulaufender Vorsprung ist mit einer Grundfläche (179) angrenzend an den Körperabschnitt und einem freien Ende (180), wobei der spitz zulaufende Vorsprung an der Grundfläche breiter ist als an dem freien Ende.

6. Spritzenvorrichtung nach Anspruch 1, wobei der ringförmige Dichtring (75, 175) ein radialer, freitragender Vorsprung (275) ist oder wobei der ringförmige Dichtring (275) zwischen zwei ringförmigen Nuten (276) positioniert ist oder wobei der ringförmige Dichtring einstückig mit dem Körperabschnitt ausgebildet ist.

7. Spritzenvorrichtung nach Anspruch 1, die ferner einen verlängerten, hohlen Nadelschutz (91) aufweist mit einem distalen Ende (92) und einem offenen proximalen Ende (93), das lösbar in den Ansatz eingreift, so dass der Nadelschutz die Kanüle bedeckt.

8. Spritzenvorrichtung nach Anspruch 1, wobei der Ansatz (68) und die Kanüle (61) integral aus thermoplastischem Material ausgebildet sind.

9. Spritzenvorrichtung nach Anspruch 1, wobei die Kanüle (96, 296) ein stumpfes distales Endteil (295) aufweist oder wobei die Kanüle aus Metall besteht.

10. Spritzenvorrichtung nach Anspruch 1, wobei das mindestens eine Gewinde (57) ein Rechtsgewinde ist und/oder wobei das mindestens eine Gewinde ein mehrgängiges Gewinde ist.

11. Spritzenvorrichtung nach Anspruch 1, wobei die Vorrichtungen zum lösbaren Eingreifen des Ansatzes in den Kragen mindestens einen Gewindeeingriffsvorsprung (78) auf dem Ansatz aufweisen oder mindestens einen Gewindeeingriffsvorsprung auf einer Innenfläche des Kragens.

12. Spritze und abnehmbare Nadelvorrichtung nach Anspruch 1, die ferner Folgendes aufweisen:
einen verlängerten, hohlen Nadelschutz (91) mit einem distalen Ende (92) und einem offenen proximalen Ende (93), das lösbar in den Ansatz eingreift, so dass der Nadelschutz die Kanüle bedeckt; und
einen verlängerten Kolben (82) mit einem proximalen Ende (83), einem distalen Ende (84) und einem Stopfen (87) am distalen Ende des Kolbens, wobei der Stopfen verschiebbar positioniert ist in fluiddichter Verbindung mit der Innenfläche des Zylinders (49), um die Flüssigkeit aus der Kammer durch die Kanüle (96) zu verdrängen mittels einer Relativbewegung des Kolbens (82) zum Zylinder (49).

## Revendications

1. Ensemble seringue et aiguille détachable (47) ayant des caractéristiques d'espace mort réduit comprenant :
un cylindre de seringue allongé (49) ayant un axe longitudinal (50), une surface interne (51) définissant une chambre (52) pour retenir un fluide, une extrémité proximale ouverte (53), une extrémité distale ouverte (55) comportant un collier (56) et une surface annulaire orientée de manière distale (58) faisant saillie vers l'intérieur à partir dudit collier, une partie dudit collier ayant une paroi latérale circulaire alignée axialement de forme cylindrique ;
un ensemble aiguille (67) comportant un raccord (68) ayant une partie de corps (69) comportant une extrémité proximale (70), une extrémité distale (71), un conduit (73) à travers celle-ci, une surface annulaire orientée de manière proximale (74) sur ladite partie de corps entrant en contact avec ladite surface annulaire dudit cylindre formant un joint d'étanchéité primaire entre ledit raccord et ledit cylindre, ledit raccord comportant en outre une bague d'étanchéité annulaire faisant saillie vers l'extérieur (75) s'engageant de manière étanche avec ladite paroi latérale circulaire dudit collier formant un joint d'étanchéité secondaire entre ledit raccord et ledit cylindre, ledit ensemble aiguille (67) comportant en outre une canule (96) ayant une extrémité distale (98), une extrémité proximale (97) et une lumière (99) à travers celle-ci, ladite extrémité proximale de ladite canule étant reliée à ladite extrémité distale dudit raccord de sorte que ladite lumière soit en communication fluidique avec ladite chambre, et
un moyen pour engager de manière amovible ledit raccord avec ledit collier comportant au moins un filetage (57) sur une surface interne (61) dudit collier ou au moins un filetage sur une surface externe dudit raccord (68),
**caractérisé en ce que**
la surface annulaire orientée de manière distale (58) du cylindre est plate et la surface annulaire orientée de manière proximale (74) du raccord est plate ;
deux saillies d'engagement de filetage (78) sur des côtés opposés du raccord (68) ;
dans lequel, pendant l'installation, la rotation du raccord (68) amènera les saillies d'engagement d'aiguille (78) du raccord (68) à suivre le filetage jusqu'à ce que la surface annulaire orientée de manière proximale (74) du raccord (68) entre en contact avec la surface annulaire orientée de manière distale (58) du cylindre (49) pour former un joint d'étanchéité primaire entre le raccord et le cylindre ;
la bague d'étanchéité annulaire faisant saillie vers l'extérieur (75) est positionnée entre les saillies filetées (78) et la surface annulaire orientée de manière proximale (74) du raccord ;
et l'ensemble aiguille est installé ou retiré du cylindre par rotation du raccord, et où le joint d'étanchéité secondaire est un joint de retenue dans le cas où l'utilisateur ne parvient pas à fournir suffisamment de couple de rotation tout en reliant le raccord au cylindre pour engager correctement le joint d'étanchéité primaire .

2. Ensemble seringue de la revendication 1, comportant en outre une tige de piston allongée (82) ayant une extrémité proximale (83), une extrémité distale (84) et un bouchon (87) au niveau de ladite extrémité distale de ladite tige de piston, ledit bouchon étant positionné en coulissement en contact étanche aux fluides avec ladite surface interne dudit cylindre pour déplacer le fluide de ladite chambre à travers ladite canule par un mouvement relatif de ladite tige de piston par rapport audit cylindre.

3. Ensemble seringue de la revendication 2, dans lequel ledit bouchon comporte une saillie orientée de manière distale (88) pour obstruer partiellement ledit conduit dans ledit raccord lorsque ledit bouchon est dans sa position la plus distale dans ledit cylindre.

4. Ensemble seringue de la revendication 2, dans lequel ladite bague d'étanchéité annulaire (75) est un joint torique élastomère et dans lequel ledit joint torique a de préférence une section transversale de forme circulaire.

5. Ensemble seringue de la revendication 1, dans lequel ladite bague d'étanchéité annulaire (75, 175) est une saillie effilée ayant une base (179) adjacente à ladite partie de corps et une extrémité libre (180), ladite saillie effilée étant plus large au niveau de ladite base qu'au niveau de ladite extrémité libre.

6. Ensemble seringue de la revendication 1, dans lequel ladite bague d'étanchéité annulaire (75, 175) est une saillie radiale en porte-à-faux (275) ou dans lequel ladite bague d'étanchéité annulaire (275) est positionnée entre deux rainures annulaires (276) ou dans lequel ladite bague d'étanchéité annulaire est formée d'un seul tenant avec ladite partie de corps.

7. Ensemble seringue de la revendication 1, comportant en outre une protection d'aiguille creuse allongée (91) ayant une extrémité distale (92) et une extrémité proximale ouverte (93) engagée de manière amovible avec ledit raccord de sorte que ladite protection d'aiguille recouvre ladite canule.

8. Ensemble seringue de la revendication 1, dans lequel ledit raccord (68) et ladite canule (96) sont formés d'un seul tenant en matériau thermoplastique.

9. Ensemble seringue de la revendication 1, dans lequel ladite canule (96, 296) comporte une pointe distale émoussée (295) ou dans lequel ladite canule est formée de métal.

10. Ensemble seringue de la revendication 1, dans lequel ledit au moins un filetage (57) est un filetage à droite et/ou dans lequel ledit au moins un filetage est un filetage multiple.

11. Ensemble seringue de la revendication 1, dans lequel ledit moyen pour engager de manière amovible ledit raccord avec ledit collier comporte au moins une saillie d'engagement de filetage (78) sur ledit raccord ou au moins une saillie d'engagement de filetage sur une surface interne dudit collier.

12. Ensemble seringue et aiguille détachable de la revendication 1, comprenant en outre :
une protection d'aiguille creuse allongée (91) ayant une extrémité distale (92) et une extrémité proximale ouverte (93) engagée de manière amovible avec ledit raccord de sorte que ladite protection d'aiguille (91) recouvre ladite canule ; et
une tige de piston allongée (82) ayant une extrémité proximale (83), une extrémité distale (84) et un bouchon (87) au niveau de ladite extrémité distale de ladite tige de piston, ledit bouchon (87) étant positionné en coulissement en contact étanche aux fluides avec ladite surface interne dudit cylindre (49) pour déplacer le fluide de ladite chambre à travers ladite canule (96) par un mouvement relatif de ladite tige de piston (82) par rapport audit cylindre (49).
